# EUROPEAN PATENT APPLICATION

(11) **EP 4 729 078 A2**
(43) Date of publication of application: **22.04.2026**
(21) Application number: 25204548.9
(22) Date of filing: 25.09.2025
(51) Int. Cl.: A61L 9/12, A61L 9/20, F24F 7/003, F24F 7/007

(54) **CLEAN STORAGE SYSTEM FOR HOME VENTILATOR**

(30) Priority: 28.09.2024 CN 202411364084
(71) Applicant: Hunan Taiyanglong Medical Technology Co., Ltd., Liuyang City, Hunan Province 410300 (CN)
(72) Inventor: CHEN, Lian, Liuyang City, 410300 (CN); CHEN, Zhiliang, Liuyang City, 410300 (CN); YANG, Xuwu, Liuyang City, 410300 (CN); CHEN, Sheng, Liuyang City, 410300 (CN)
(74) Representative: Chung, Hoi Kan

(57) **Abstract**

Disclosed is a clean storage system for a home ventilator, including a body with a ventilator placement region, storage device, air purification system, and display control component. Air outlets are at least formed in the ventilator placement region or a portion of the body proximal to the ventilator placement region. The storage device is provided with a storage chamber, exhaust ports, and a storage opening being opened or closed. The storage chamber communicates with an air supply device. The air purification system is provided with a fan, an air purification device, the air outlets, and air inlets. An airflow channel in an air outflow direction of the air purification device in the body is a clean air region, and the air outlets communicate with the airflow channel, such that a clean air area is formed in the ventilator placement region, and the air supply device draws in clean air.

## Description

### TECHNICAL FIELD

The present disclosure relates to a clean storage system for a home ventilator, and belongs to the field of cleaning and disinfection equipment.

### BACKGROUND

Sleep apnoea syndrome has become a relatively common sleep respiratory disorder. Its main clinical manifestations are snoring during sleep and daytime sleepiness. It not only seriously affects sleep quality, but also easily leads to hypertension, diabetes, and cardiovascular and cerebrovascular diseases in a long term. In severe cases, it can even cause sudden death during sleep, making it a potentially fatal sleep respiratory disorder.

Continuous positive pressure ventilation through a home ventilator of a non-invasive ventilation structure during sleep is an effective means for treating sleep apnoea syndrome. Especially for cases caused by lung failure or obstructive sleep apnoea, the use of a ventilator can effectively improve the cases as it does not require hospital surgery or drug treatment. Its efficacy and convenience are increasingly accepted by a large number of patients. However, during the use of the home ventilator, there are still some problems and inconvenience in the storage of accessories/components of the home ventilator and use environments.

Firstly, as a medical electrical appliance, the home ventilator requires a safe and fixed platform region for placement to ensure reliable operation. However, daily usage scenarios of the home ventilator are mainly bedrooms, and patients are accustomed to placing the home ventilator on bedside tables, which leads to the mixture of the home ventilator and their accessories with other daily necessities, easily interfering with and contaminating operating environments of the home ventilator.

Secondly, during the use, a mouth and nose mask, a nose mask, and a nose plug arranged on the home ventilator directly make contact with a patient's face. Grease seeping from skin and bacteria and viruses produced by exhalation through the mouth and nose can accumulate and breed therein. Condensate water generated by a respiratory connecting tube and water stains in a humidification box are very likely to breed bacteria and viruses. To ensure healthy use, the home ventilator should be cleaned and disinfected in time and stored cleanly for later use after every use. However, the above conditions are not achieved in daily home storage. Therefore, even if the home ventilator is completely cleaned by a user, the above accessories/components will still be contaminated again during the storage process.

In addition, in terms of the use environments, due to the limitations of the power and structure of an air feed device, only a single layer of primary/ medium-efficiency filter cotton is arranged for an air source for simple filtration. This will cause dust particles smaller than 5 µm, including bacteria and viruses in air, to be simultaneously inhaled into the home ventilator and enter the human body through airways, leading to viral infection in the lungs. Moreover, dust particles, bacteria and viruses that enter airways and various components of the home ventilator will accumulate and breed. Affected by air pollution, the air filter cotton needs to be replaced in time every half a month to a month. Due to the influence of user's personal habits, replacement time is bound to be highly uncertain and inconvenient. Objectively, there is also a certain degree of health risk.

### SUMMARY

As for the above technical problems, the present disclosure provides a clean storage system for a home ventilator. The clean storage system for the home ventilator provides a placement space for various types of home ventilators, has functions of achieving clean storage of accessories/components of the home ventilator and filtering and purifying an air source, so as to ensure operation and cleanliness of the air source required for the home ventilator, and is capable of purifying air in indoor environments.

The objectives of the present disclosure are achieved by the following technical solution:

A clean storage system for a home ventilator is characterized by including a body with a ventilator placement region, a storage device, an air purification system, and a display control component, where
air outlets are at least formed in the ventilator placement region or a portion of the body proximal to the ventilator placement region;
the storage device is provided with a storage chamber, exhaust ports, and a storage opening capable of being opened or closed, and the storage chamber communicates with an air supply device;
the air purification system is provided with a fan, an air purification device, the air outlets, and air inlets; and
an airflow channel in an air outflow direction of the air purification device in the body is a clean air region, and the air outlets communicate with the airflow channel, such that the clean air region is formed in the ventilator placement region, and the air supply device draws in clean air.

In the technical solution of the present disclosure, the body further includes an air tube cleaning connecting portion, the air tube cleaning connecting portion communicates with the airflow channel, and the air tube cleaning connecting portion is provided with a connecting portion movable cover.

In the technical solution of the present disclosure, the body further includes an air tube cleaning connecting portion, the air supply device communicates with the air tube cleaning connecting portion, the clean air drawn in by the air supply device is respectively conveyed to the storage chamber and the air tube cleaning connecting portion, and the air tube cleaning connecting portion is provided with a connecting portion movable cover.

In the technical solution of the present disclosure, an ozone generator is arranged in an air outflow direction of the air supply device.

In the technical solution of the present disclosure, the air supply device has a heating structure, which draws in the clean air, and heats and then conveys the same.

In the technical solution of the present disclosure, the ventilator placement region is formed by a concave structure with at least one opening in the body, and the air outlets are formed in the concave structure.

In the technical solution of the present disclosure, the ventilator placement region is formed by a concave structure on a top of the body, and the air outlets are formed in convex portions at a bottom end and/or periphery of the ventilator placement region.

In the technical solution of the present disclosure, the ventilator placement region is formed by a concave structure on a side portion of the body, and the air outlets are formed in a bottom end and/or side of the ventilator placement region.

In the technical solution of the present disclosure, the ventilator placement region is formed by a platen fixedly or movably connected to a side portion or upper end of the body, the air outlets are formed in the platen or a portion, on the platen, of the body, and the movable connection includes coupling, articulated arm connection and sliding connection.

In the technical solution of the present disclosure, the platen is connected to the upper end of the body through a rotary shaft, and the platen and the rotary shaft are both provided with the airflow channel.

In the technical solution of the present disclosure, the ventilator placement region is provided with a movable protective cover, and the movable protective cover is provided with the air outlets and/or a pipeline connector.

In the technical solution of the present disclosure, the storage chamber is further provided with a storage and placement component and an opening or closing door or movable cover sealing the storage opening, the storage and placement component is a storage plate or storage basket formed by lattices/grids and fixedly or movably mounted in the storage chamber, and a sealing structure is arranged at an end, in contact with the movable cover or the opening or closing door, of the storage opening.

In the technical solution of the present disclosure, the storage opening is formed in a side portion of the storage chamber and sealed by the coupled opening or closing door, and the storage and placement component is a storage plate.

In the technical solution of the present disclosure, the storage opening is formed in an upper end of the storage chamber and connected and sealed by the detachable or coupled movable cover, and the storage and placement component is a storage plate.

In the technical solution of the present disclosure, the storage opening is formed in a side portion of the storage chamber, the storage and placement component is a storage basket, the storage basket and the opening or closing door are connected to be movably connected to the storage chamber through a pull structure, and the storage opening is sealed by the opening or closing door.

In the technical solution of the present disclosure, the storage chamber is further provided with at least one supporting element and/or at least one sterilization and disinfection device, and the supporting elements bear, suspend, and fix accessories of the home ventilator through at least one contact point or contact surface.

In the technical solution of the present disclosure, the sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure.

In the technical solution of the present disclosure, the air purification device is composed of one or at least two of an activated carbon filter, a cartridge filter, an anion purifier, a photocatalyst purifier, and a plasma purifier.

In the technical solution of the present disclosure, the activated carbon filter and the cartridge filter are of a barrel-shaped structure or a platy structure.

In the technical solution of the present disclosure, the activated carbon filter and/or the cartridge filter of the platy structure is connected to the body by a drawer type pull structure.

In the technical solution of the present disclosure, the activated carbon filter and/or the cartridge filter of the barrel-shaped or platy structure is connected to the body by an embedding structure, and a movable plate is detachably connected or coupled to a portion of the body corresponding to the activated carbon filter and/or the cartridge filter.

In the technical solution of the present disclosure, the cartridge filter is composed of one or at least two of a primary filter, a medium-efficiency filter, and a high-efficiency filter.

In the technical solution of the present disclosure, the body is provided with movable wheels.

In the technical solution of the present disclosure, the body is further provided with a power socket and/or a USB interface.

The technical solution of the present disclosure further provides a clean storage system for a home ventilator, characterized by including a body with a ventilator placement region, a storage device, an air purification system, and a display control component, where
air outlets are at least formed in the ventilator placement region or a portion of the body proximal to the ventilator placement region;
the storage device is provided with a storage chamber, exhaust ports, and a storage opening capable of being opened or closed, the storage chamber communicates with an air supply device, and the air supply device draws in external air of the body;
the air purification system is provided with a fan, an air purification device, the air outlets, and air inlets; and
an airflow channel in an air outflow direction of the air purification device in the body is a clean air region, and the air outlets communicate with the airflow channel, such that the clean air region is formed in the ventilator placement region.

The present disclosure has the following advantages and beneficial effects:
1. The ventilator placement region suitable for placing various brands and types of home ventilators is arranged, placement convenience is provided for use of the home ventilator, and the problem of the lack of a specific placement platform for the home ventilator for daily use is solved. The local clean air region can be formed in the placement region, such that the home ventilator placed therein is located in a clean air environment, and provides a clean air source required for ventilation therapy.
2. The body is provided with components and spaces for storage of the accessories/components of the home ventilator, a mouth and nose mask/nose mask/nose plug, a respiratory air tube and a humidification box thereof can be stored therein after being used daily or cleaned and then air-dried/dried, and the clean air is used for airdrying/drying and can be continuously conveyed to the storage chamber, such that the storage chamber can be kept in a clean state, thereby meeting the requirements of clean storage of the above accessories/components stored therein and keeping sanitary safety of daily use.
3. The air purification device is arranged in the body. Through more efficient filtration structures such as a high-efficiency filter and an activated carbon filter in the air purification device, dust particles smaller than 5 µm, as well as bacteria and viruses, are filtered and purified, thereby providing the clean air source for air-drying/drying and the home ventilator. This not only can improve the quality of air required for ventilation and clean storage, but also can ensure the cleanliness of various components and airways in the home ventilator, eliminating the need to clean internal structures and an air path channel of the home ventilator. Especially for users with pulmonary failure and other conditions that require higher air quality, it also eliminates the risk of patients' lung infection caused by insufficient air filtration. The service life and replacement cycle of the air purification device are longer. No frequent replacement or maintenance is required, thereby achieving more convenient use, and the service life of a filtration device of the home ventilator is further improved.
4. By the adoption of the air purification device, a function of purifying air in indoor environments is achieved, thereby achieving multiple purposes.

### BRIEF DESCRIPTION OF THE DRAWINGS

FIG. 1 is a schematic structural diagram of Embodiment 1 of the present disclosure.
FIG. 2 is a schematic structural diagram of a direction A in FIG. 1.
FIG. 3 is a schematic structural diagram of a section cutting plane of a line B-B in FIG. 2.
FIG. 4 is a schematic structural diagram of a section cutting plane of a line C-C in FIG. 2.
FIG. 5 is a schematic diagram of an airflow flow direction in an operating state where Embodiment 1 of the present disclosure and a home ventilator are used in cooperation.
FIG. 6 is a schematic diagram of an airflow flow direction in an operating state for cleaning a storage chamber and an air tube of Embodiment 1 of the present disclosure.
FIG. 7 is a schematic diagram of a clean air conveying frame of the present disclosure.
FIG. 8 is a schematic structural diagram of Embodiment 2 of the present disclosure.
FIG. 9 is a schematic structural diagram of a section cutting plane of a line D-D in FIG. 8.
FIG. 10 is a schematic diagram of an airflow flow direction in an operating state where Embodiment 2 of the present disclosure and a home ventilator are used in cooperation.
FIG. 11 is a schematic diagram of an airflow flow direction in an operating state for cleaning a storage chamber and an air tube of Embodiment 2 of the present disclosure.
FIG. 12 is a schematic structural diagram of Embodiment 3 of the present disclosure.
FIG. 13 is a schematic structural diagram of a direction E in FIG. 12.
FIG. 14 is a schematic structural diagram of a section cutting plane of a line F-F in FIG. 13.
FIG. 15 is a schematic structural diagram of a section cutting plane of a line G-G in FIG. 13.
FIG. 16 is a schematic diagram of an airflow flow direction in an operating state where Embodiment 3 of the present disclosure and a home ventilator are used in cooperation.
FIG. 17 is a schematic diagram of an airflow flow direction in an operating state for cleaning a storage chamber and an air tube of Embodiment 3 of the present disclosure.
FIG. 18 is a partial sectional schematic structural diagram in a rotating state of a rotary platen of Embodiment 3 of the present disclosure.
FIG. 19 is a schematic diagram in an opened state of a movable protective cover of Embodiment 3 of the present disclosure.
FIG. 20 is a schematic structural diagram of Embodiment 4 of the present disclosure.
FIG. 21 is a schematic diagram of an airflow flow direction in an operating state where Embodiment 4 of the present disclosure and a home ventilator are used in cooperation.

Reference signs: 1-body, 2-opening or closing door, 3-exhaust port, 4-ventilator placement region, 5-display control component, 6-connecting portion movable cover, 7-air outlet, 8-storage chamber, 9-fan, 10-air purification device, 11-air inlet, 12-airflow channel, 13-ozone generator, 14-air tube, 15-air tube cleaning connecting portion, 16-storage and placement component, 17-air supply device, 18-air chamber, 19-home ventilator, 20-respiratory air tube, 21-respiratory mask, 22-movable cover, 23-rotary platen, 24-movable protective cover, 25-rotary shaft, 26-airflow communication port, 27-pipeline connector, and 28-overturning platen.

### DETAILED DESCRIPTION OF THE EMBODIMENTS

The present disclosure will be described in detail below with reference to accompanying drawings and specific embodiments.

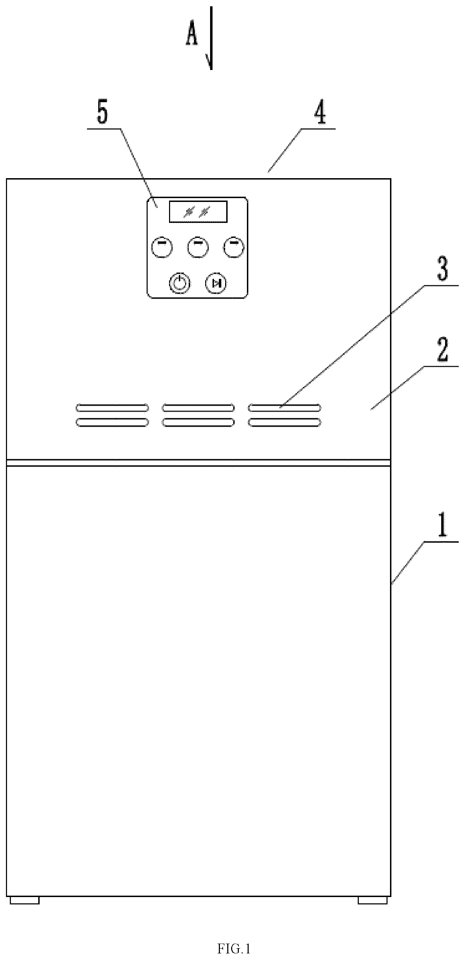

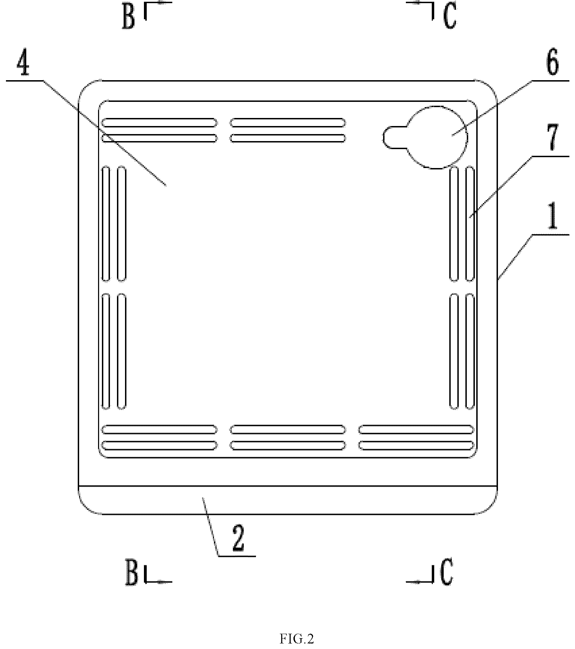

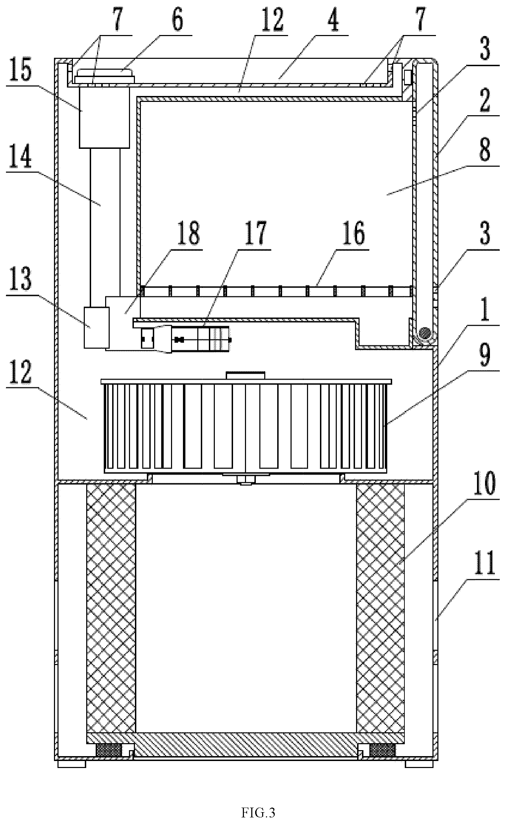

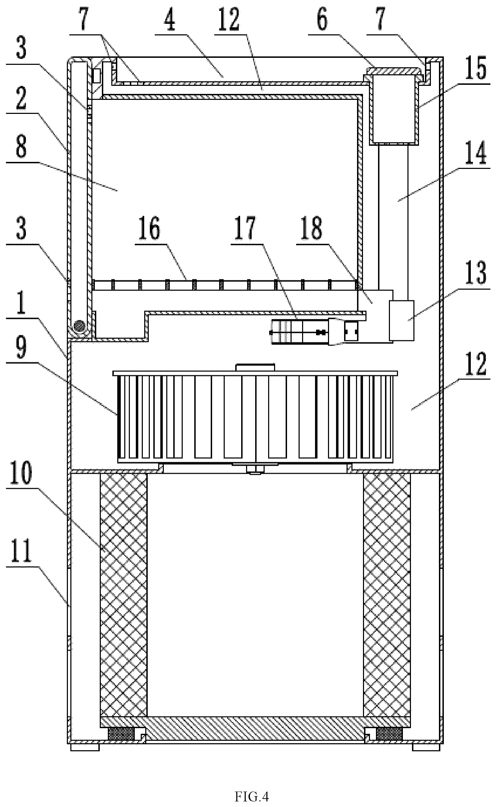

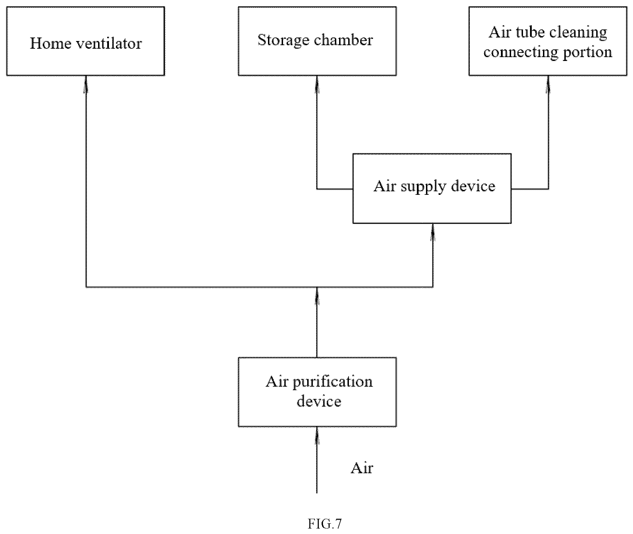

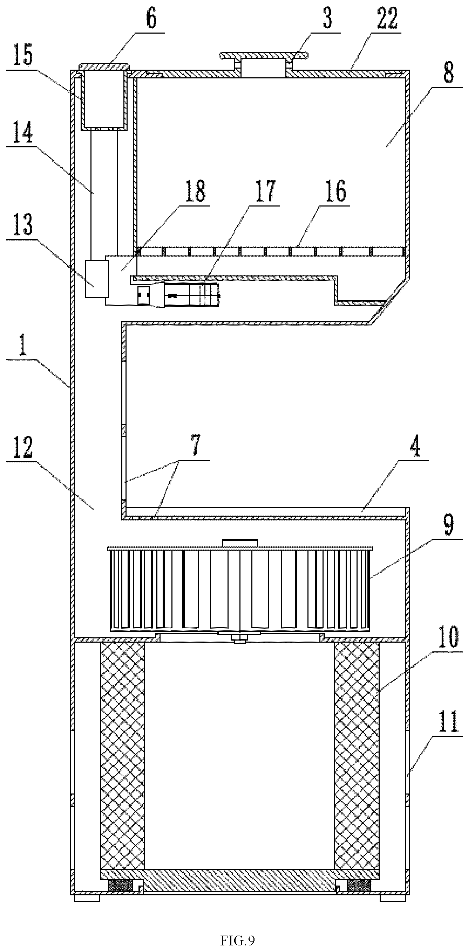

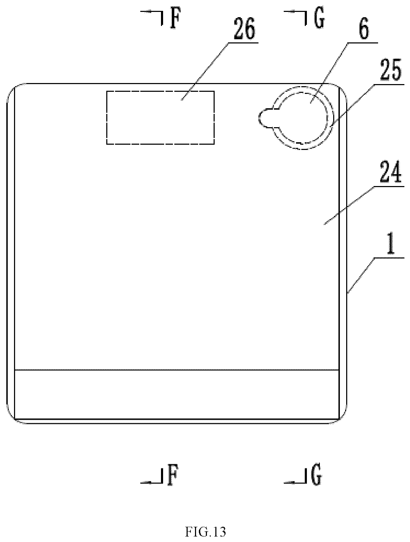

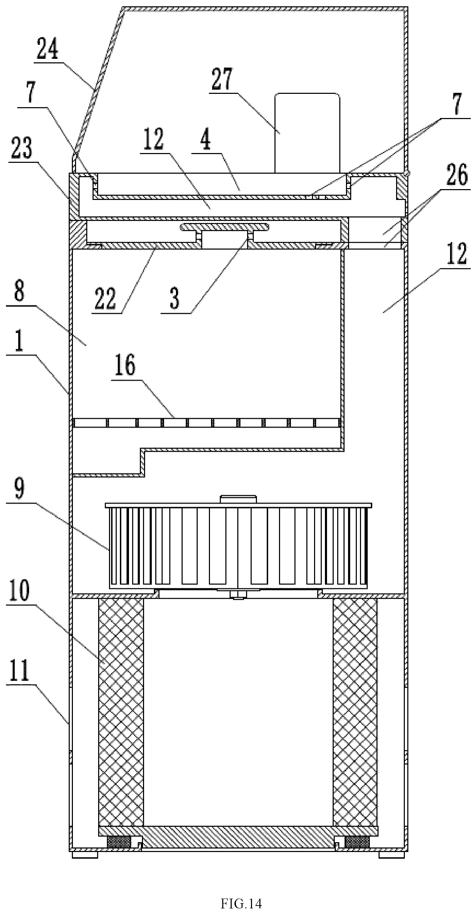

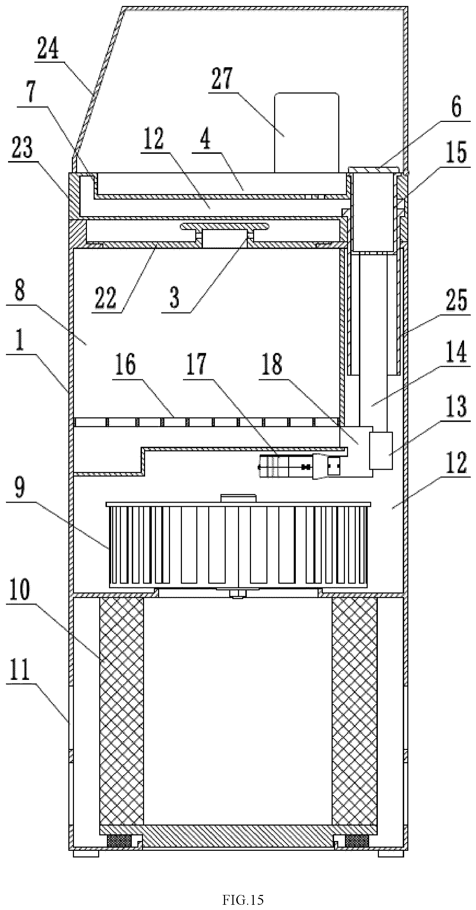

FIG. 1, FIG. 2, FIG. 3, FIG. 4, FIG. 5, FIG. 6, FIG. 7, FIG. 8, FIG. 9, FIG. 10, FIG. 11, FIG. 12, FIG. 13, FIG. 14, FIG. 15, FIG. 16, FIG. 17, FIG. 18, FIG. 19, FIG. 20, and FIG. 21 illustrate embodiments of the present disclosure. A clean storage system for a home ventilator is formed by assembling a body 1, an opening or closing door 2, exhaust ports 3, a ventilator placement region 4, a display control component 5, a connecting portion movable cover 6, air outlets 7, a storage chamber 8, a fan 9, an air purification device 10, air inlets 11, an airflow channel 12, an ozone generator 13, an air tube 14, an air tube cleaning connecting portion 15, a storage and placement component 16, an air supply device 17, an air chamber 18, a home ventilator 19, a respiratory air tube 20, a respiratory mask 21, a movable cover 22, a rotary platen 23, a movable protective cover 24, a rotary shaft 25, airflow communication ports 26, a pipeline connector 27, an overturning platen 28, connecting components, and fasteners.

Specific embodiments have the following structural characteristics.

A clean storage system for a home ventilator is characterized by including a body 1 with a ventilator placement region 4, a storage device, an air purification system, and a display control component 5, where
air outlets 7 are at least formed in the ventilator placement region 4 or a portion of the body 1 proximal to the ventilator placement region 4;
the storage device is provided with a storage chamber 8, exhaust ports 3, and a storage opening capable of being opened or closed, and the storage chamber 8 communicates with an air supply device 17;
the air purification system is provided with a fan 9, an air purification device 10, the air outlets 7, and air inlets 11; and
an airflow channel 12 in an air outflow direction of the air purification device 10 in the body 1 is a clean air region, and the air outlets 7 communicate with the airflow channel 12, such that the clean air region is formed in the ventilator placement region 4, and the air supply device 17 draws in clean air.

In the embodiment of the present disclosure, the external air is introduced into the body 1 through the air inlets 11 via negative pressure produced by the fan 9, and filtered and purified by the air purification device 10 into the clean air. The airflow channel 12 is formed by the clean air flow region in the air outflow direction of the air purification device 10 in the body 1, and the clean air is exhausted out of the body 1 through the air outlets 7. The air outlets 7 may be formed in the ventilator placement region 4, such that the clean air region is formed in the ventilator placement region 4, the home ventilator 19 placed in the ventilator placement region 4 draws in the clean air in the region to serve as an air source required for ventilation therapy, the air supply device 17 draws in the clean air as an air source required for air-drying/drying the storage chamber 8, a function of clean storage can be achieved while environment air is purified, cleanliness of the air source for ventilation therapy is improved, and cleanliness of internal components and an air path channel of the home ventilator 19 are ensured.

In some embodiments, the air outlets 7 may also be formed in the airflow channel 12 in the clean air region in the body 1 besides the ventilator placement region 4, so as to further improve purification efficiency of the environment air.

In the embodiment of the present disclosure, the ventilator placement region 4 may be arranged on an adaptive portion of the body 1, such as an upper end, upper portion, middle portion or lower portion of the body 1. Accessories/components of the home ventilator 19 are stored in the storage chamber 8, including but not limited to a respiratory mask 21, a nose mask, a nose plug, a respiratory air tube 20, and a humidification box.

In some embodiments, the body 1 may be provided with an air tube cleaning connecting portion 15, the air tube cleaning connecting portion 15 communicates with the airflow channel 12 to draw in the clean air, the respiratory air tube 20 can be connected to the air tube cleaning connecting portion 15 after being used or cleaned, so as to be air-dried or only suspended, and a connecting portion movable cover 6 is arranged on the air tube cleaning connecting portion 15, so as to achieve a protective effect in a non-use state.

In some embodiments, for example, the body 1 is provided with an air tube cleaning connecting portion 15 and a connecting portion movable cover 6, and the air tube cleaning connecting portion 15 communicates with the air supply device 17, such that the clean air drawn in by the air supply device 17 is respectively conveyed to the storage chamber 8 and the air tube cleaning connecting portion 15, and the clean air is also conveyed to the air tube cleaning connecting portion 15 while being conveyed to the storage chamber 8 by the air supply device 17.

In the embodiment of the present disclosure, the air supply device 17 is provided with an air feed driving component and an air intake vent, and the air supply device 17 may communicate with any applicable position such as a bottom, a side face or an upper portion of the storage chamber 8.

In some embodiments, an ozone generator 13 may be arranged in an air outflow direction of the air supply device 17, such that ozone can be conveyed to the storage chamber 8 by the air supply device 17 to perform ozone sterilization and disinfection on the storage chamber 8 and the accessories/components of the home ventilator stored therein. In the embodiment where the air supply device communicates with the air tube cleaning connecting portion 15, the air supply device 17 further can convey the ozone to the respiratory air tube 20 connected with the air tube cleaning connecting portion 15 to perform ozone sterilization and disinfection on the respiratory air tube.

In some embodiments, the air supply device 17 may have a heating structure, and the heating structure can heat the clean air drawn in by the air supply device 17 and then convey the same to the storage chamber 8, such that the accessories/components of the home ventilator stored in the storage chamber 8 are dried and sterilized and disinfected at a high temperature. In the embodiment where the air supply device communicates with the air tube cleaning connecting portion 15, the air supply device 17 further can convey the heated clean air to the respiratory air tube 20 connected with the air tube cleaning connecting portion 15 to perform dry and sterilize and disinfect the respiratory air tube at a high temperature.

In the embodiment of the present disclosure, the ventilator placement region 4 is formed by a concave structure with at least one opening in the body 1, and the air outlets 7 are formed in the concave structure. It is to be understood that the concave structure may be formed in a top end of the body 1 or a side portion of the body 1. In a case where the concave structure is formed at the top end of the body 1, the air outlets 7 are formed in convex portions at a bottom end and/or periphery of the ventilator placement region 4. In a case where the concave structure is formed on the side portion of the body 1, the air outlets 7 are formed in the bottom end and/or side of the ventilator placement region 4.

In some other embodiments, the ventilator placement region 4 is formed by a platen fixedly or movably connected to a side portion or top end of the body 1, the air outlets 7 are formed in the platen or a portion, on the platen, of the body 1, and the movable connection includes coupling, articulated arm connection and sliding connection. For example, a rotary platen 23 is connected to the top end of the body 1 through a rotary shaft 25, and the rotary platen 23 and the rotary shaft 25 are both provided with the airflow channel 12. For another example, an overturning platen 28 is coupled to one side portion of the body 1.

In some other embodiments, the platen further can be slidably connected to the upper end of the body 1, and the air outlets 7 communicating with the airflow channel 12 are formed in the platen.

In the embodiment of the present disclosure, the ventilator placement region 4 is provided with a movable protective cover 24. For example, in a case where the body 1 is the respiratory placement region 4 formed by the concave structure, the movable protective cover 24 is arranged on the opening of the concave structure. In a case where the ventilator placement region 4 is formed by the platen, the movable protective cover 24 is arranged on the platen. Due to the arrangement of the movable protective cover 24, the home ventilator 19 placed in the ventilator placement region 4 can be protected (fall prevention, dust prevention, etc.), and a better clean air region can be formed in the ventilator placement region 4. The movable protective cover 24 is provided with the air outlets 7 and/or a pipeline connector 27.

In the embodiment of the present disclosure, the air purification device 10 is composed of one or at least two of an activated carbon filter, a cartridge filter, an anion purifier, a photocatalyst purifier, and a plasma purifier.

That is, the air purification device 10 may be composed of one of the above structures or may be composed of two or more of the above structures. In a case where the air purification device adopts two air purification structures, air is sequentially filtered and purified by the two different purification structures, so as to achieve a better air purification effect.

In some embodiments, the air purification device 10 is composed of two structures, namely the activated carbon filter and the cartridge filter. Air can be purified by the cartridge filter and then secondarily purified by the activated carbon filter, or the air is purified by the activated carbon filter and then secondarily purified by the cartridge filter. A sequence of the two air purification structures can be determined as required according to positions where they are mounted on an airflow path. The same is true for the embodiments adopting other purifier structures.

In some embodiments, the cartridge filter is composed of one or a combination of at least two of a primary filter, a medium-efficiency filter, and a high-efficiency filter. That is, the cartridge filter may be composed of one of the above structures, or may be composed of a combination of two or more of the above structures.

For example, in some embodiments, the primary filter, the medium-efficiency filter, and the high-efficiency filter can be combined to form the cartridge filter.

In some embodiments, in a case where two air purification devices 10 are arranged, one is a cartridge filter composed of the primary filter, and the other is a cartridge filter composed of the high-efficiency filter.

It is to be understood that a specific structure and connecting and mounting manners of the air purification device 10 are not exhaustive in the embodiment of the present disclosure. For example, an air purification device of a barrel-shaped structure can be applied to the embodiment of the present disclosure as long as it is an air purification structure used for relevant high-quality products available on the market or existing air purifiers.

The activated carbon filter, the cartridge filter, the anion purifier, the photocatalyst purifier, the plasma purifier, the primary filter, the medium-efficiency filter, and the high-efficiency filter are all the prior art and are relevant high-quality products available on the market, and their specific connecting structures are not repeated here, which should be known to those skilled in the art.

As for various air purification structures adopted by the air purification device 10 in the embodiment of the present disclosure, as existing mature arts, whether they can filter particulate matter, bacteria, allergens, etc., filtration and purification effects they can achieve are all reflected in test reports and product introductions of various products available on the market. Therefore, compared with the prior art, the clean air filtered and purified by the air purification device 10 as the air source used for the home ventilator 19, drying, high-temperature disinfection, and clean storage is significantly improved.

In some embodiments, the air purification device 10 is embedded into the body 1 through, for example, a barrel-shaped or platy structure, and a detachably-connected or coupled movable plate may be mounted on the portion of the body 1, such that the air purification device 10 can be conveniently maintained and replaced.

In the embodiment of the present disclosure, the storage chamber 8 is further provided with a storage and placement component 16 and the storage opening, the storage and placement component 16 is a storage plate or storage basket formed by lattices/grids and mounted in the storage chamber 8 through a fixed or detachable structure, and the storage opening can be sealed through the opening or closing door 2 or the movable cover 22 according to different arrangement portions of the storage opening of the storage chamber 8.

In the embodiment of the present disclosure, a sealing structure is arranged on a portion, in contact with the storage opening of the storage chamber 8, of the opening or closing door 2 or the movable cover 22, which may be a staggered sealing structure or a rubber sealing ring.

In some embodiments, in a case where the storage opening is, for example, formed in the upper end of the storage chamber 8, one of the movable cover 22 or the opening or closing door 2 may be adopted. In a case where the storage opening is formed in one side portion of the storage chamber 8, the opening or closing door 2 may be adopted. In these embodiments, the movable cover 22 is coupled or detachably connected to the storage chamber 8, the opening or closing door 2 is coupled to the storage chamber 8, and the storage and placement component 16 may adopt the storage plate. Of course, the storage and placement component and the opening or closing door 2 may be connected into a pull basket structure to be movably connected to the storage chamber 8 as required, or the storage and placement component 16 is of a pull basket structure while the opening or closing door 2 adopts a coupled opening or closing structure.

In the embodiment of the present disclosure, at least one supporting element may be arranged at a lower end, upper end or side face of the storage chamber 8 or may be arranged on the storage and placement component 16, and the supporting elements bear, suspend, and fix the accessories/components of the home ventilator 19 through at least one contact point or contact surface. The contact points or contact surfaces adaptive to structures of the accessories/components of the home ventilator 19 are formed by the arrangement number and structures of the supporting elements, for example, in a case where arc-shaped structures are arranged in the accessories/components, the contact surfaces therebetween can be adaptive through the supporting elements of the arc-shaped structures, or contact points therebetween are adaptive through two or more supporting elements of columnar structures in a multipoint contact manner. It can be understood that the supporting elements may be arranged into a rod shape or a hook shape, such that the accessories of the home ventilator 19 can be stably borne, suspended and fixed.

In some embodiments, the storage chamber 8 is further provided with a sterilization and disinfection device, such that the storage chamber 8 and the accessories/components of the home ventilator 19 stored therein can be conveniently sterilized and disinfected, thereby further improving cleanliness of space environments of the storage chamber 8 and the stored accessories/components.

In the embodiment where the sterilization and disinfection device is arranged, the sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure.

For example, in some embodiments, the storage chamber 8 is provided with one sterilization and disinfection device, which is the UV ultraviolet sterilization and disinfection structure. In some other embodiments, the storage chamber 8 is provided with two sterilization and disinfection devices, namely the UV ultraviolet sterilization and disinfection structure and the plasma sterilization and disinfection structure. In the embodiment where the two sterilization and disinfection devices are mounted, two identical sterilization and disinfection structures may also be adopted, for example, both the two sterilization and disinfection devices adopt the UV ultraviolet sterilization and disinfection structure.

In some embodiments, the body 1 may be provided with movable wheels, such that the clean storage system can be conveniently moved by a user to different positions for use.

In some embodiments, the body 1 may further be provided with a power socket and/or a USB interface, such that the home ventilator 19 can be powered on and used nearby.

In the embodiment of the present disclosure, when the respiratory mask 21, the humidification box, and the respiratory air tube 20 are placed in the storage chamber 8 after being cleaned, the display control component 5 and corresponding control modules control the fan 12, the air supply device 17, the air purification device 10, the ozone generator 13, and the sterilization and disinfection device to be started or stopped regularly. The clean air or heated cleaned air is intermittently or continuously conveyed to the storage chamber 8 or the ozone generator 13 and a disinfection medium of the sterilization and disinfection device to perform clean storage on the respiratory mask 21, the humidification box, and the respiratory air tube 20. The display control component 5 includes functional keys and a display device. The control modules include module devices achieving the above functions.

In the embodiment of the present disclosure, the display control component 5 and the control modules are all the prior general art and are relevant high-quality products available on the market, which should be known to those skilled in the art, and their specific connecting structures are not repeated here.

FIG. 1, FIG. 2, FIG. 3, FIG. 4, FIG. 5, FIG. 6, and FIG. 7 illustrate Embodiment 1 of the present disclosure.

In Embodiment 1, an air purification device 10 and a fan 9 are sequentially arranged on a lower portion of an inner cavity of a body 1 from bottom to top, the air purification device 10 is of a barrel-shaped structure and embedded into the body 1, and a plurality of air inlets 11 are formed in a portion, on the air purification device 10, of the body 1.

A storage chamber 8 is arranged above the fan 9, a storage opening is formed in a side portion of the storage chamber 8, the storage opening is opened or closed through a coupled opening or closing door 2, an airflow cavity is formed in the opening or closing door 2, exhaust ports 3 are respectively formed in an inner side face and an outer side face of the opening or closing door 2, a storage and placement component 16 formed by a storage plate is mounted on a lower portion of the storage chamber 8, and an air outflow direction of an air supply device 17 communicates with a lower end of the storage chamber 8 through an air chamber 18.

A ventilator placement region 4 is formed by a concave structure at a top end of the body 1, and a plurality of air outlets 7 are formed in a bottom end peripheral portion and convex portions of the ventilator placement region 4 of the concave structure.

An air tube cleaning connecting portion 15 is arranged at the top end of the body 1, the air tube cleaning connecting portion 15 communicates with the air chamber 18 through an air tube 14, and the air tube cleaning connecting portion 15 is provided with a connecting portion movable cover 6.

The air supply device 17 has a heating structure, and the air chamber 18 is provided with an ozone generator 13.

A display control component 5 is embedded into an upper portion of the body 1. The display control component 5 includes a display device and control keys corresponding to clean storage functions. The above functions are achieved by control modules arranged in the inner cavity of the body 1. The control modules are respectively connected with the display control component 5, the fan 9, the air supply device 17, and the ozone generator 13, and receive, process, and send corresponding information to control the above functional components to work.

An air outflow direction of the air purification device 10 is an airflow channel 12 for clean air to flow, and an air intake vent and air outlets 7 of the air supply device 17 respectively communicate with the airflow channel 12, such that the air supply device 17 draws in the clean air, and meanwhile a local clean air region is formed in the ventilator placement region 4.

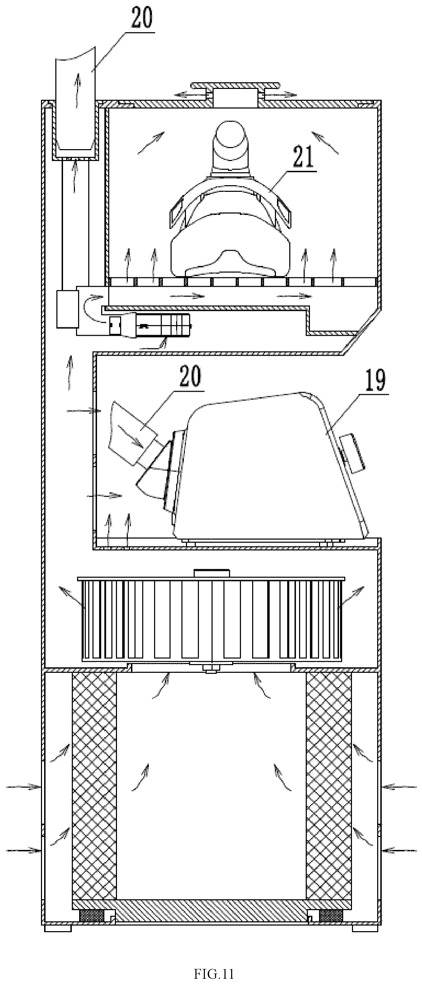

Referring to a schematic diagram of a clean air conveying frame shown in FIG. 11, the clean air filtered and purified by the air purification device 10 is respectively conveyed to the home ventilator 19 and the air supply device 17, and the clean air is respectively conveyed to the storage chamber 8 and the air tube cleaning connecting portion 15 after being heated by the air supply device 17.

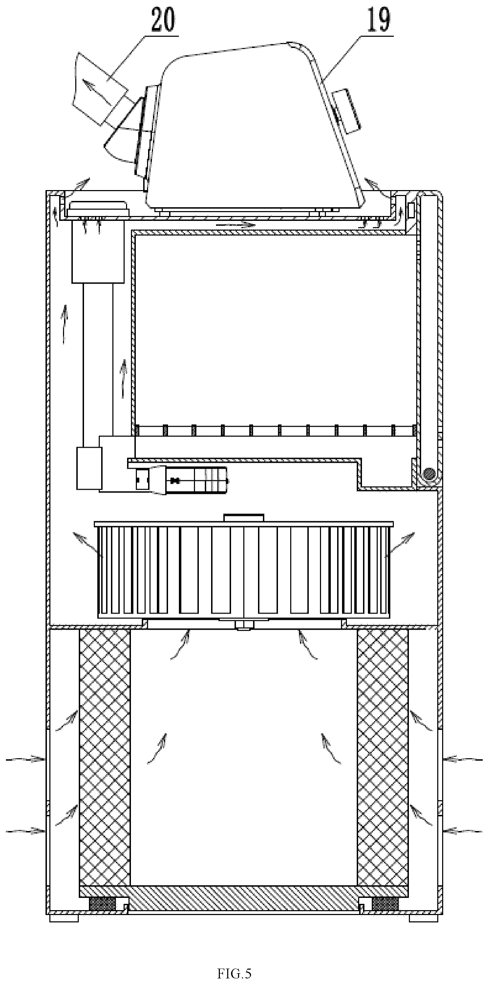

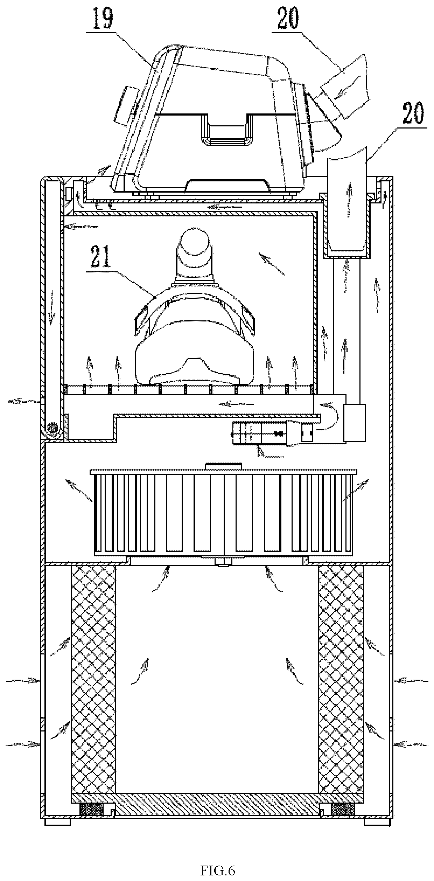

FIG. 5 and FIG. 6 illustrate an airflow flow direction in an operating state of Embodiment 1.

Referring to FIG. 5, FIG. 5 illustrates an airflow flow direction in an operating state where Embodiment 1 and the home ventilator 19 are used in cooperation. When a patient uses the home ventilator 19 for ventilation therapy, the home ventilator 19 is placed in the ventilator placement region 4, namely the concave structure at the top end of the body 1 in Embodiment 1, the fan 9 is started to produce negative pressure airflow, the external air of the body 1 enters the inner cavity of the body 1 from the air inlets 11, after the external air is filtered and purified by the air purification device 10, the airflow channel 12 for the clean air to flow is formed in the inner cavity of the body 1 in an air outflow direction of the fan 9, the clean air is output from the air outlets 7 through the airflow channel 12, at this moment, the clean air region is formed above the home ventilator placement region 4, an air inflow portion of the home ventilator 19 placed therein will directly draw in the clean air in the region to serve as an air source required for ventilation therapy, and the clean air exhausted from the air outlets 7 will be simultaneously conveyed into indoor environments.

Referring to FIG. 6, FIG. 6 illustrates an airflow flow direction in an operating state for drying the respiratory mask 21 and the respiratory air tube 20 of Embodiment 1.

After the patient cleans or uses the respiratory mask 21 and places the same in the storage chamber 8, the fan 9 is started to produce negative pressure airflow, the external air of the body 1 enters the inner cavity of the body 1 from the air inlets 11, after being filtered and purified by the air purification device 10, the air enters the airflow channel 12 in the air outflow direction of the fan 9, the air supply device 17 is started to draw in the clean air in the airflow channel 12 and heats the same, the heated clean air is conveyed to the storage chamber 8 through the air chamber 18 to dry the respiratory mask 21, and the clean air and water vapor in the storage chamber 8 are exhausted from the exhaust ports 3 in the opening or closing door 2.

When the respiratory air tube 20 is dried, one end of the respiratory air tube 20 is connected with the air tube cleaning connecting portion 15, and the other end of the respiratory air tube is connected with the respiratory air tube connector of the home ventilator 19. In a case where the humidification box is detached, the clean air heated by the air supply device 17 is continuously conveyed to the respiratory air tube 20 through the air tube and the air tube cleaning connecting portion 15, and meanwhile exhausted out through the respiratory air tube connector and a connecting airway of the home ventilator 19 and the humidification box. During this process, the other end of the respiratory air tube 20 may not be connected with the respiratory tube connector, and at this time, the heated clean air for drying the respiratory air tube is exhausted out from the other end of the respiratory air tube.

It is to be noted that when the heated clean air is conveyed to the storage chamber 8 and the air tube cleaning connecting portion 15, the ozone generator 13 may be started as required, which can perform ozone sterilization and disinfection on an air draw-in tube 37, the storage chamber 8 and the accessories/components stored in the storage chamber, the ozone generator 13 may be independently used in a non-drying state as required, and it is only necessary to control start and stop of the air supply device 17.

After the respiratory mask 21 and the respiratory air tube 20 are dried, the display control component 5 and the corresponding control modules regularly control start and stop of the fan 9 and the air supply device 17, the heated clean air is intermittently or continuously conveyed to the storage chamber 8 and the air tube cleaning connecting portion 15, so as to achieve the clean storage function of the accessories/components of the home ventilator such as the respiratory mask 21 and the respiratory air tube 20.

It is to be noted that the heating structure of the air supply device 17 can heat the clean air to a required suitable temperature, and the air supply device 17 can output the heated clean air at a certain temperature accordingly, so as to meet the requirements of drying, high-temperature sterilization and disinfection, and clean storage.

It is to be noted that the air supply device 17 and the heating structure of the air supply device 17 are both relevant high-quality products available on the market. As the prior art, their specific structures are not repeated here.

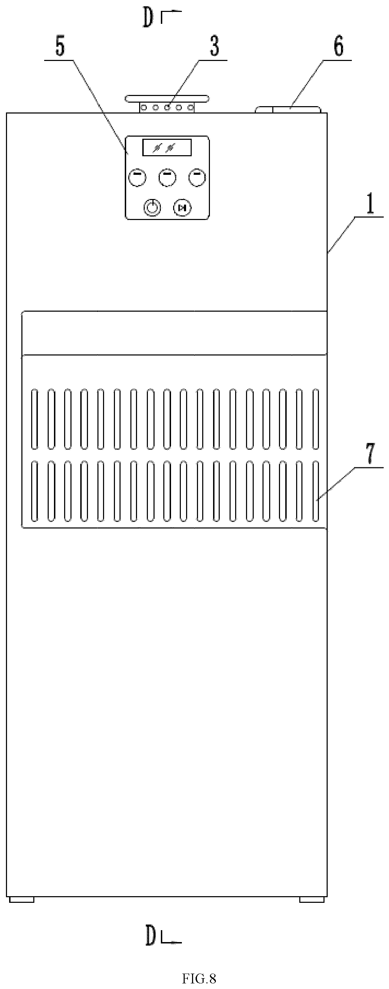

FIG. 8, FIG. 9, FIG. 10, and FIG. 11 illustrate Embodiment 2 of the present disclosure. The parts of this embodiment that are the same as those of Embodiment 1 are not repeated here. In this embodiment, a ventilator placement region 4 is formed by a concave structure in a middle of a body 1, and the concave structure is provided with two openings formed in a front direction and a right direction respectively. Air outlets 7 communicating with an airflow channel 12 are formed in a bottom end and a side portion of the concave structure, clean air is conveyed to the concave structure through the air outlets 7, a local clean air region is formed by the concave structure accordingly, a storage opening of a storage chamber 8 is formed in an upper end, the opening of the storage chamber 8 is sealed by a movable cover 22, and exhaust ports 3 are formed in the movable cover 22.

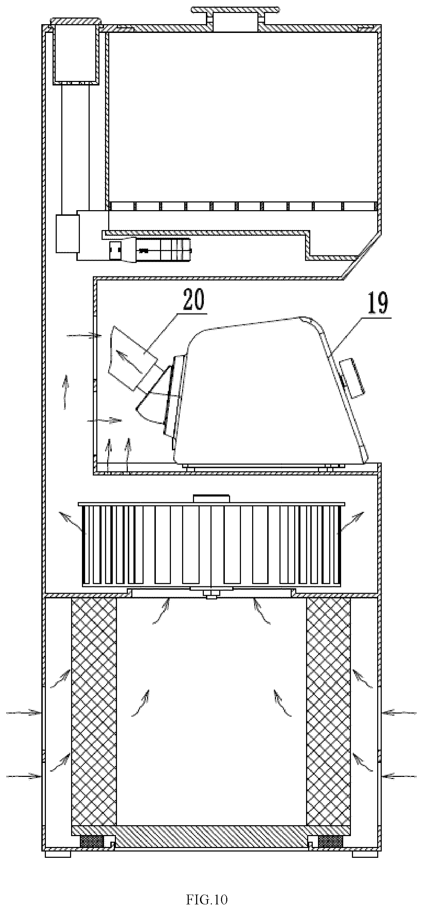

FIG. 10 illustrates an airflow flow direction in an operating state where Embodiment 2 and a home ventilator 19 are used in cooperation.

When a patient uses the home ventilator 19 for ventilation therapy, the home ventilator 19 is placed in the concave structure, a fan 9 is started to produce negative pressure airflow, external air of the body 1 enters an inner cavity of the body 1 from air inlets 11, after the external air is filtered and purified by an air purification device 10, the airflow channel 12 for the clean air to flow is formed in the inner cavity of the body 1 in an air outflow direction of the fan 9, the clean air is output to the concave structure from the air outlets 7 through the airflow channel 12, an air inflow portion of the home ventilator 19 placed therein will directly draw in the clean air as an air source required for ventilation therapy, and the clean air exhausted from the air outlets 7 will be simultaneously conveyed into indoor environments.

FIG. 11 illustrates an airflow flow direction in an operating state for drying a respiratory mask 21 and a respiratory air tube 20 of Embodiment 2.

This embodiment is the same as Embodiment 1 except that the clean air and water vapor in the storage chamber 8 are exhausted out from the exhaust ports 3 of the movable cover 22, which is not repeated here.

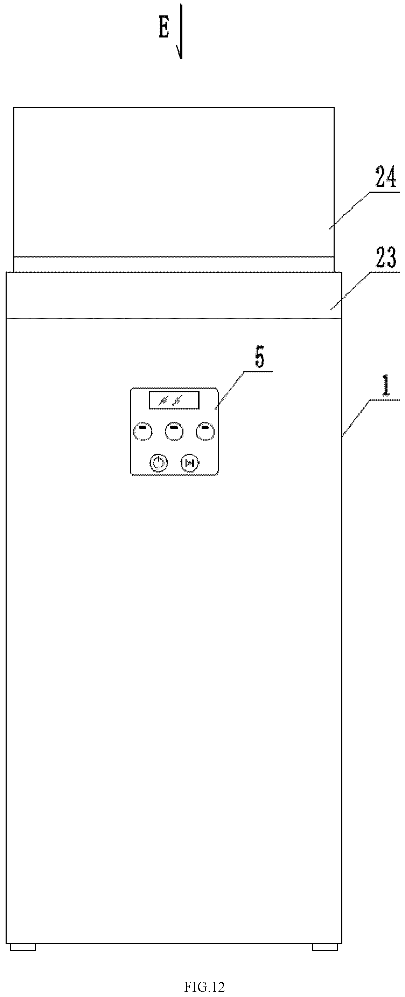

FIG. 12, FIG. 13, FIG. 14, FIG. 15, FIG. 16, FIG. 17, FIG. 18, and FIG. 19 illustrate Embodiment 3 of the present disclosure. The parts of this embodiment that are the same as those of Embodiment 1 are not repeated here. In this embodiment, a ventilator placement region 4 is formed by a rotary platen 23, which horizontally rotates along a top end of a body 1 through a rotary shaft 25, the rotary platen 23 is provided with airflow channels 12, air outlets 7 and the ventilator placement region 4 formed by a concave structure are arranged on the rotary platen 23, and the rotary platen 23 and the body 1 are provided with corresponding airflow communication ports 26. The body 1 communicates with the respectively airflow channels 12 of the rotary platen 23 through the airflow communication ports 26, such that clean air can be output to a position above the rotary platen 23 from the air outlets 7 to form a clean air region. An air tube cleaning connecting portion 15 sleeved with the rotary shaft 25 and communicating with an air chamber 18 through an air tube 14 is arranged on the rotary platen 23, and the air tube cleaning connecting portion 15 is provided with a connecting portion movable cover 6. The rotary platen 23 is further provided with a movable protective cover capable of being opened in an overturned manner, a pipeline connector 27 and air outlets 7 are formed in the movable protective cover 24, the clean air in the ventilator placement region 4 can be exhausted out of the movable protective cover 24 from the pipeline connector 27 and the air outlets 7, the storage opening of the storage chamber 8 is located in an upper end thereof, namely below the rotary platen 23, the opening of the storage chamber 8 is sealed by a movable cover 22, and exhaust ports 3 are formed in the movable cover 22.

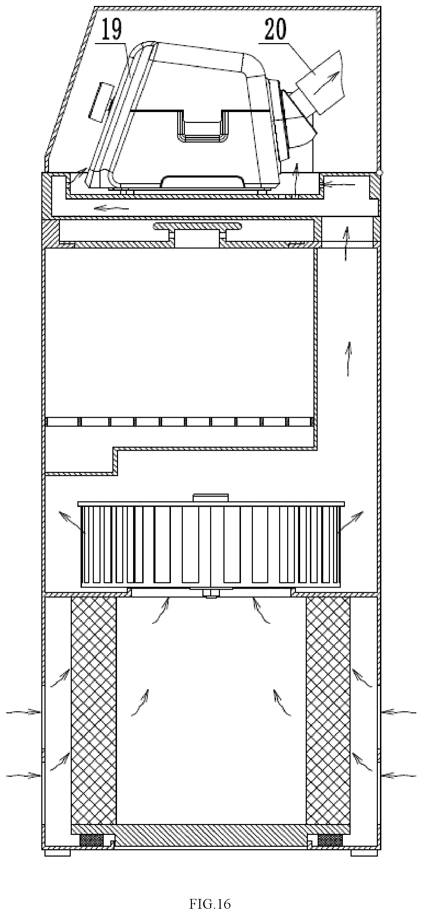

FIG. 16 illustrates an airflow flow direction in an operating state where Embodiment 3 and a home ventilator 19 are used in cooperation.

When a patient uses the home ventilator 19 for ventilation therapy, the movable protective cover 24 is opened, the home ventilator 19 is placed in the ventilator placement region 4 of the rotary platen 23, the movable protective cover 24 is closed, a fan 9 is started to produce negative pressure airflow, external air of the body 1 enters an inner cavity of the body 1 from air inlets 11, after the external air is filtered and purified by an air purification device 10, the airflow channels 12 for the clean air to flow are formed in the inner cavity of the body 1 in an air outflow direction of the fan 9, the clean air enters the airflow channels 12 of the rotary platen 23 through the airflow communication ports 26 formed between the rotary platen 23 and the body 1 and output from the air outlets 7 formed in the rotary platen 23, a local clean air region is formed between the rotary platen 23 and the movable protective cover 24 accordingly, and the clean air in the region is output to environment air from the air outlets 7 formed in the movable protective cover 24. The air outlets 7 are not shown in the figure, however, it is to be understood that the air outlets 7 may be formed in any position of the movable protective cover 24, as long as they can output the clean air to environments.

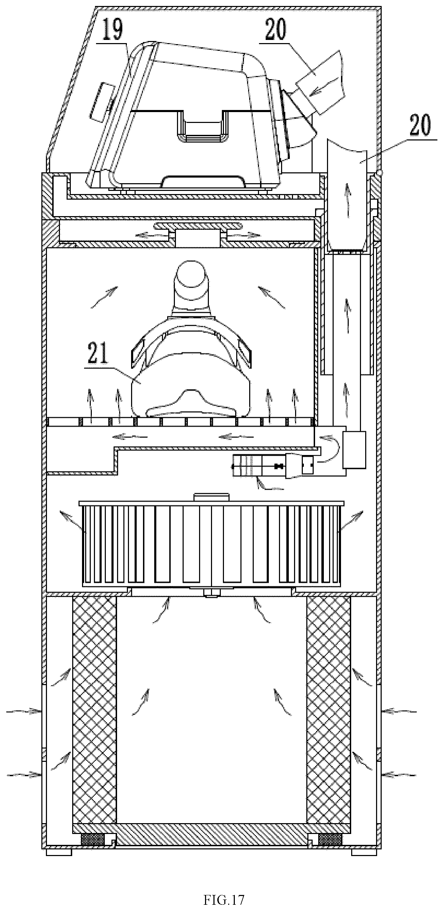

FIG. 17 illustrates an airflow flow direction in an operating state for drying a respiratory mask 21 and a respiratory air tube 20 of Embodiment 3.

In this embodiment, the airflow flow direction in the operating state for drying the respiratory mask 21 is the same as that in Embodiment 2, which is not repeated here. The airflow flow direction in the operating state for drying the respiratory air tube 20 is the same as that in Embodiment 1, which is not repeated here.

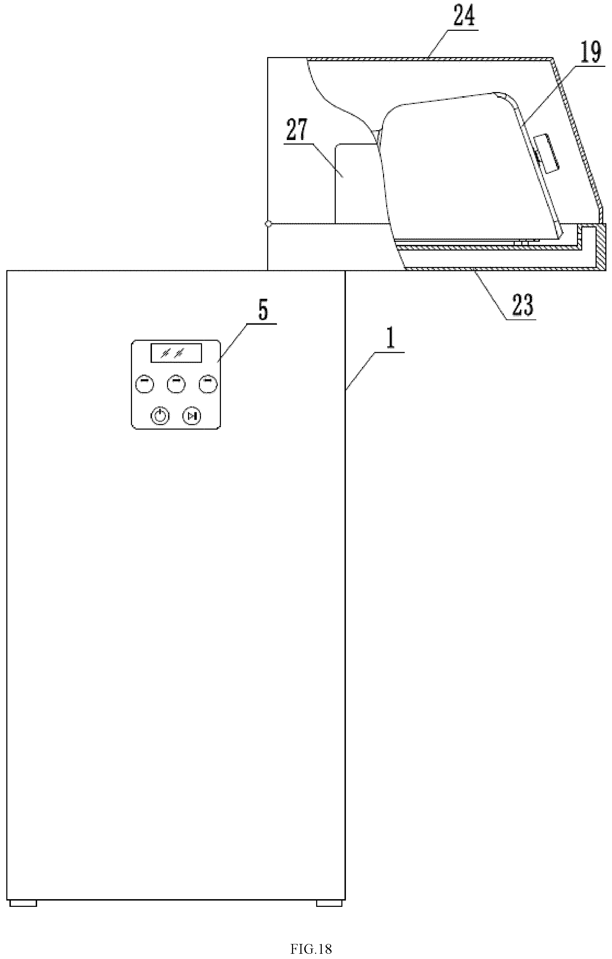

FIG. 18 illustrates that in Embodiment 3, the rotary platen 23 rotates to a certain angle state, when it is necessary to open the movable cover 22 at the upper end of the storage chamber 8, the rotary platen 23 needs to rotate to a certain angle, and horizontal rotary displacement of the rotary platen 23 can further provide an expansion function with a wider use range for the home ventilator 19 placed therein besides for opening the movable cover 22 of the storage chamber 8.

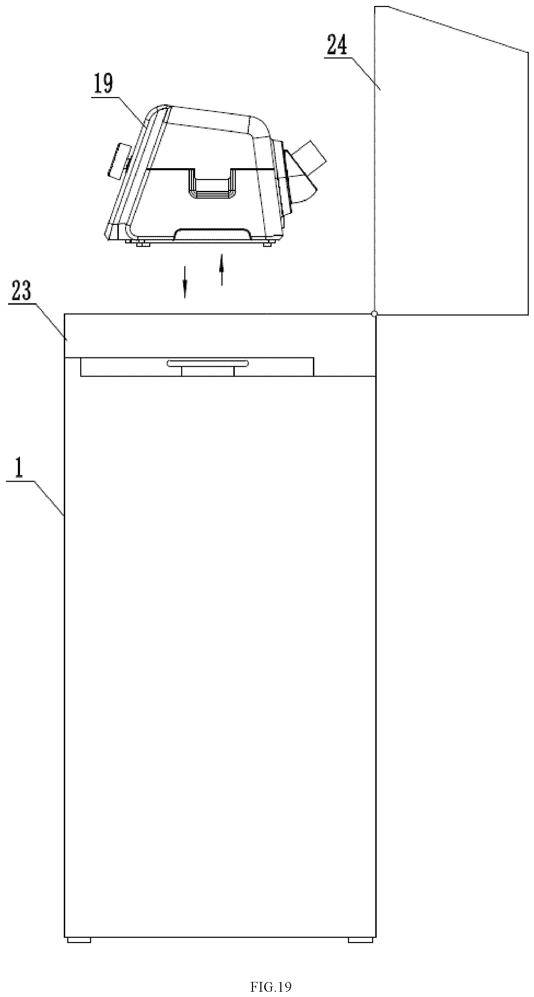

FIG. 19 illustrates a state where the movable protective cover 24 is opened for placing and taking out of the home ventilator 19 in Embodiment 3.

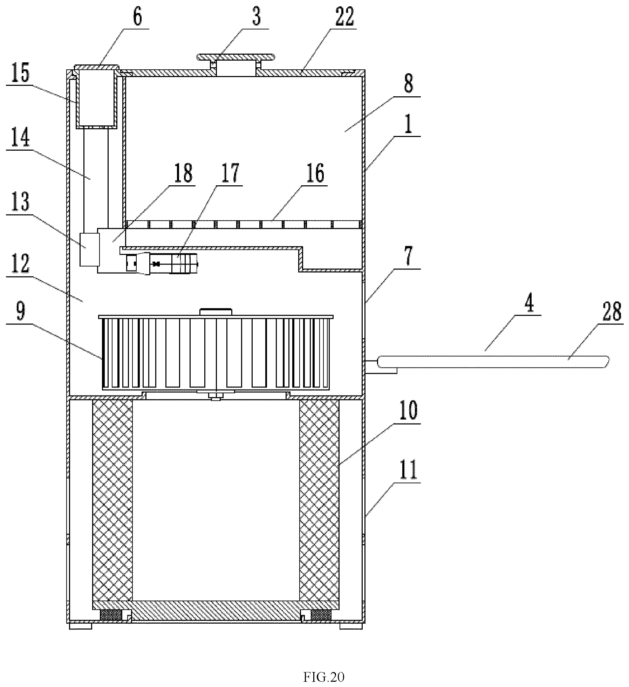

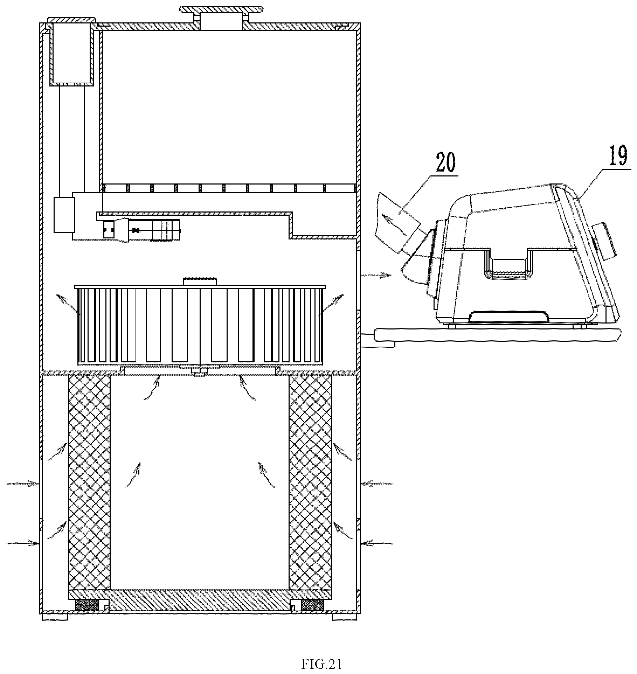

FIG. 20 and FIG. 21 illustrate Embodiment 4 of the present disclosure. The parts of this embodiment that are the same as those of Embodiment 1 are not repeated. In this embodiment, a ventilator placement region 4 is formed by an overturning platen 28 coupled to a side of a body 1, air outlets 7 are formed in a portion, proximal to the overturning platen 28 and above the same, of the body 1, clean air is conveyed to a platen region from the air outlets 7, a clean air region is formed in the platen region, a storage opening of a storage chamber 8 is located in an upper end thereof, the opening of the storage chamber 8 is sealed by a movable cover 22, and exhaust ports 3 are formed in the movable cover 22.

FIG. 21 illustrates an airflow flow direction in an operating state where Embodiment 4 and a home ventilator 19 are used in cooperation.

When a patient uses the home ventilator 19 for ventilation therapy, the home ventilator 19 is placed on the overturning platen 28, a fan 9 is started to produce negative pressure airflow, external air of the body 1 enters an inner cavity of the body 1 from air inlets 11, after the external air is filtered and purified by an air purification device 10, an airflow channel 12 for the clean air to flow is formed in the inner cavity of the body 1 in an air outflow direction of the fan 9, the clean air is output to the overturning platen 28 from the air outlets 7 through the airflow channel 12, an air inflow portion of the home ventilator 19 placed therein will draw in the clean air in the region of the overturning platen 28 to serve as an air source required for ventilation therapy, and the clean air exhausted from the air outlets 7 will be simultaneously conveyed into indoor environments.

Airflow flow directions in an operating state for drying a respiratory mask 21 and a respiratory air tube 20 in this embodiment are both the same as those in the above embodiments, which are not repeated here.

In some embodiments of the present disclosure, an air intake vent of an air supply device 17 communicates with an exterior of the body 1, such that the air supply device directly draws in external air of the body 1 to serve as an air source for air-drying/drying, and other component settings and specific structures in addition to this may adopt the contents in Embodiments 1, 2, 3, and 4.

It is to be noted that in the embodiment of the present disclosure, the air supply device 17 and the heating structure of the air supply device 17 are both relevant high-quality products available on the market. As the prior art, their specific structures are not repeated here.

It is to be noted that in the embodiment of the present disclosure, the UV ultraviolet sterilization and disinfection structure, the plasma sterilization and disinfection structure, the photocatalyst sterilization and disinfection structure, and the ozone sterilization and disinfection structure (ozone generator 13) are all the prior general art and are relevant high-quality products available on the market, which should be known to those skilled in the art, and their specific connecting structures are not repeated here.

It is to be noted that in the embodiment of the present disclosure, better purposes of drying and sterilization and disinfection can be achieved by controlling a certain temperature of the heating structure of the air supply device 17, please refer to pasteurism and dry heat sterilization for sterilization and disinfection principles and required temperatures.

It is to be understood that although the present disclosure mainly aims to solve the problems about daily home use scenes, it does not mean that it cannot be used by a user in other places (such as medical care, rehabilitation, health care and other places with needs) after purchase.

It is finally illustrated that although the above embodiments have described the technical solution of the present disclosure in detail, they cannot be regarded as all embodiments included in the technical solution of the present disclosure, and there are replacements, variations and equivalent embodiments that fall within the scope of the technical solution of the present disclosure. Those ordinarily skilled in the art should understand that other means with multiple methods and equipment for implementing the present disclosure should be understood as including all the substitutions, variations and equivalent embodiments that fall within the spirit and scope of the present disclosure.

## Claims

1. A clean storage system for a home ventilator, **characterized by** comprising a body (1) with a ventilator placement region (4), a storage device, an air purification system, and a display control component (5), wherein
air outlets (7) are at least formed in the ventilator placement region or a portion of the body proximal to the ventilator placement region;
the storage device is provided with a storage chamber (8), exhaust ports (3), and a storage opening capable of being opened or closed, and the storage chamber communicates with an air supply device (17);
the air purification system is provided with a fan (9), an air purification device (10), the air outlets (7), and air inlets (11); and
an airflow channel (12) in an air outflow direction of the air purification device in the body is a clean air region, and the air outlets communicate with the airflow channel, such that a clean air area is formed in the ventilator placement region, and the air supply device draws in clean air.

2. The clean storage system for the home ventilator according to claim 1, **characterized in that** the body (1) further comprises an air tube cleaning connecting portion (15), the air tube cleaning connecting portion communicates with the airflow channel (12), and the air tube cleaning connecting portion is provided with a connecting portion movable cover (6).

3. The clean storage system for the home ventilator according to claim 1, **characterized in that** the body (1) further comprises an air tube cleaning connecting portion (15), the air supply device (17) communicates with the air tube cleaning connecting portion, the clean air drawn in by the air supply device is respectively conveyed to the storage chamber (8) and the air tube cleaning connecting portion, and the air tube cleaning connecting portion is provided with a connecting portion movable cover (6).

4. The clean storage system for the home ventilator according to claim 1 or 3, **characterized in that** an ozone generator (13) is arranged in an air outflow direction of the air supply device (17).

5. The clean storage system for the home ventilator according to claim 1 or 3, **characterized in that** the air supply device (17) has a heating structure, which draws in the clean air, and heats and then conveys the same.

6. The clean storage system for the home ventilator according to claim 1, **characterized in that** the ventilator placement region (4) is formed by a concave structure with at least one opening in the body (1), and the air outlets (7) are formed in the concave structure.

7. The clean storage system for the home ventilator according to claim 6, **characterized in that** the ventilator placement region (4) is formed by a concave structure on a top of the body (1), and the air outlets (7) are formed in convex portions at a bottom end and/or periphery of the ventilator placement region.

8. The clean storage system for the home ventilator according to claim 6, **characterized in that** the ventilator placement region (4) is formed by a concave structure on a side portion of the body (1), and the air outlets (7) are formed in a bottom end and/or side of the ventilator placement region.

9. The clean storage system for the home ventilator according to claim 1, **characterized in that** the ventilator placement region (4) is formed by a platen fixedly or movably connected to a side portion or upper end of the body (1), the air outlets (7) are formed in the platen or a portion, on the platen, of the body, and the movable connection comprises coupling, articulated arm connection and sliding connection.

10. The clean storage system for the home ventilator according to claim 9, **characterized in that** the platen is connected to the upper end of the body (1) through a rotary shaft (25), and the platen and the rotary shaft are both provided with the airflow channel (12).

11. The clean storage system for the home ventilator according to any one of claims 6, 7, 8, and 9, **characterized in that** the ventilator placement region (4) is provided with a movable protective cover (24), and the movable protective cover is provided with the air outlets (7) and/or a pipeline connector (27).

12. The clean storage system for the home ventilator according to claim 1, **characterized in that** the storage chamber (8) is further provided with a storage and placement component (16) and an opening or closing door (2) or movable cover (22) sealing the storage opening, the storage and placement component is a storage plate or storage basket formed by lattices/grids and fixedly or movably mounted in the storage chamber, and a sealing structure is arranged at an end, in contact with the movable cover or the opening or closing door, of the storage opening.

13. The clean storage system for the home ventilator according to claim 12, characterized in thatthe storage opening is formed in a side portion of the storage chamber (8) and sealed by the coupled opening or closing door (2), and the storage and placement component (16) is a storage plate.

14. The clean storage system for the home ventilator according to claim 12, **characterized in that** the storage opening is formed in an upper end of the storage chamber (8) and connected and sealed by the detachable or coupled movable cover (22), and the storage and placement component (16) is a storage plate.

15. The clean storage system for the home ventilator according to claim 12, **characterized in that** the storage opening is formed in a side portion of the storage chamber (8), the storage and placement component (16) is the storage basket, the storage basket and the opening or closing door (2) are connected to be movably connected to the storage chamber through a pull structure, and the storage opening is sealed by the opening or closing door.

16. The clean storage system for the home ventilator according to any one of claims 1, 3, 12, 13, 14, and 15, **characterized in that** the storage chamber (8) is further provided with at least one supporting element and/or at least one sterilization and disinfection device, and the supporting elements bear, suspend, and fix accessories of the home ventilator (19) through at least one contact point or contact surface.

17. The clean storage system for the home ventilator according to claim 16, **characterized in that** the sterilization and disinfection device is composed of one or a combination of at least two of a UV ultraviolet sterilization and disinfection structure, a plasma sterilization and disinfection structure, a photocatalyst sterilization and disinfection structure, and an ozone sterilization and disinfection structure.

18. The clean storage system for the home ventilator according to claim 1, **characterized in that** the air purification device (10) is composed of one or at least two of an activated carbon filter, a cartridge filter, an anion purifier, a photocatalyst purifier, and a plasma purifier.

19. The clean storage system for the home ventilator according to claim 18, **characterized in that** the activated carbon filter and the cartridge filter are of a barrel-shaped structure or a platy structure.

20. The clean storage system for the home ventilator according to claim 19, **characterized in that** the activated carbon filter and/or the cartridge filter of the platy structure is connected to the body (1) by a drawer type pull structure.

21. The clean storage system for the home ventilator according to claim 19, **characterized in that** the activated carbon filter and/or the cartridge filter of the barrel-shaped or platy structure is connected to the body (1) by an embedding structure, and a movable plate is detachably connected or coupled to a portion of the body corresponding to the activated carbon filter and/or the cartridge filter.

22. The clean storage system for the home ventilator according to any one of claims 18, 19, 20, and 21, **characterized in that** the cartridge filter is composed of one or at least two of a primary filter, a medium-efficiency filter, and a high-efficiency filter.

23. The clean storage system for the home ventilator according to any one of claims 1, 2, 3, 6, 7, 8, 9, 10, 20, and 21, **characterized in that** the body (1) is provided with movable wheels.

24. The clean storage system for the home ventilator according to any one of claims 1, 2, 3, 6, 7, 8, 9, 10, 20, and 21, **characterized in that** the body (1) is further provided with a power socket and/or a USB interface.

25. A clean storage system for a home ventilator, **characterized by** comprising a body (1) with a ventilator placement region (4), a storage device, an air purification system, and a display control component (5), wherein
air outlets (7) are at least formed in the ventilator placement region or a portion of the body proximal to the ventilator placement region;
the storage device is provided with a storage chamber (8), exhaust ports (3), and a storage opening capable of being opened or closed, the storage chamber communicates with an air supply device (17), and the air supply device draws in external air of the body;
the air purification system is provided with a fan (9), an air purification device (10), the air outlets (7), and air inlets (11); and
an airflow channel (12) in an air outflow direction of the air purification device in the body is a clean air region, and the air outlets communicate with the airflow channel, such that a clean air area is formed in the ventilator placement region.
